Europäisches Patentamt

(19) European Patent Office

Office européen des brevets

(11) **EP 0 720 975 A1**

(12) **EUROPEAN PATENT APPLICATION**

(43) Date of publication:
10.07.1996 Bulletin 1996/28

(51) Int. Cl.⁶: $C07C\ 17/154$, $C07C\ 19/03$, $B01J\ 23/83$

(21) Application number: 96300056.7

(22) Date of filing: 03.01.1996

(84) Designated Contracting States:
DE FR GB

(30) Priority: 06.01.1995 US 369228

(71) Applicant: **DOW CORNING CORPORATION**
**Midland Michigan 48686-0994 (US)**

(72) Inventors:
• **Crum, Bruce R.**
  **Madison, Indiana 47250 (US)**

• **Jarvis, Robert F., Jr.**
  **Union, Kentucky 41091 (US)**
• **Naasz, Brian M.**
  **DeWitt, Michigan 48820 (US)**
• **Toupadakis, Andreas I.**
  **GR-74100 Rethymno, Crete (GR)**

(74) Representative: **Dowden, Marina**
  **Elkington and Fife**
  **Prospect House,**
  **8 Pembroke Road**
  **Sevenoaks, Kent TN13 1XR (GB)**

(54) **Oxyhydrochlorination catalyst and process**

(57)    A catalyst composition and a process are described for the oxyhydrochlorination of methane to form methyl chloride. The process comprises contacting methane, oxygen and hydrogen chloride with a fixed-bed of a catalyst comprising a support selected from alumina or pyrogenic silica and, retained on the support, from 0.3 to 5 weight percent copper, from 5 to 25 weight percent lanthanum and from 0.1 to 5 weight percent lithium. The contact of the reactants with the fixed-bed of catalyst is effected at a temperature within a range of 200°C. to 400°C. This process can be conducted at a gauge pressure within a range of 0 to 689.5 kPa (0 to 100 psi).

EP 0 720 975 A1

## Description

The present invention provides a catalyst and a process for the oxyhydrochlorination of methane to form methyl chloride. The process comprises contacting methane, oxygen and hydrogen chloride with a fixed-bed of a catalyst comprising a support selected from alumina or pyrogenic silica and, retained on the support, 0.3 to 5 weight percent copper, 5 to 25 weight percent lanthanum and 0.1 to 5 weight percent lithium. The contact of the reactants with the fixed-bed of catalyst is effected at a temperature within a range of 200°C. to 400°C. The process is conducted at a gauge pressure within a range of 0 to 689.5 kPa (0 to 100 psi).

Methyl chloride is an important reactive intermediate in the organic chemical and petroleum industries as well as in the silicone industry. Standard commercial methods for producing methyl chloride involve the reaction of methanol with hydrogen chloride. In theory, methyl chloride can be made more cost effectively by an oxyhydrochlorination process. However, for reasons to be discussed below, oxyhydrochlorination is not generally employed as a commercial process for the production of methyl chloride.

The term "oxyhydrochlorination" as used herein refers to a reaction in which the source of chlorine employed for the chlorination reaction is gaseous hydrogen chloride which is made to give up its chlorine in useful form through a well known series of reactions involving oxygen and copper chlorides or the chlorides of other metals of variable valence.

The chemistry of the oxyhydrochlorination of methane to form methyl chloride is a combination of chlorination and oxidative chemistries described by the general reaction

$$CH_4 + 1/2\ O_2 + HCl \text{---> } CH_3Cl + H_2O.$$

In addition to the formation of methyl chloride, there are other potential chlorination reactions that may occur, the primary one being

$$CH_3Cl + 1/2\ O_2 + HCl \text{--> } CH_2Cl_2 + H_2O,$$

$$CH_2Cl_2 + 1/2\ O_2 + HCl \text{--> } CHCl_3 + H_2O \text{ and}$$

$$CHCl_3 + 1/2\ O_2 + HCl \text{--> } CCl_4 + H_2O.$$

The thermodynamics of this chemistry dictate the formation of the equilibrium favored $CCl_4$ byproduct. In addition, kinetic measurements show that the kinetics of chlorination favor a fast succession of chlorination steps to the more chlorinated chloromethanes.

Another series of byproduct reactions that compete with the formation of the chlorinated products are the combustion reactions that can occur. The key combustion reactions are

$$CH_4 + O_2 \text{---> } CO + 2H_2O$$

$$CH_4 + 3/2\ O_2 \text{--> } CO_2 + 2H_2O$$

$$CH_3Cl + O_2 \text{--> } CO + HCl + H_2O$$

$$CH_3Cl + 3/2\ O_2 \text{--> } CO_2 + HCl + H_2O$$

It is known that the activation energies for the combustion reactions are significantly higher than those for the chlorination reactions. Therefore, higher temperatures in a oxyhydrochlorination reaction will favor the combustion species. Furthermore, the heats of reaction for the combustion reactions are significantly higher than those for the chlorination reactions. Therefore, not only do the combustion products represent a loss of methane, but the highly exothermic formation of CO and $CO_2$ can lead to a runaway reaction. Because of the reaction energies and heats of reactions for the chemistries of the oxyhydrochlorination process, it is desirable to run the reactor under as near isothermal conditions as possible and at as low a temperature as possible to minimize the formation of oxidation products.

The challenge to providing a commercially successful oxyhydrochlorination process is to provide a process which is selective for the production of methyl chloride over more chlorinated species and which also minimizes the combustion reactions.

It is well known in the art that copper chloride can be a catalyst for the oxyhydrochlorination of methane to form methyl chloride. However, the conversion of methane to methyl chloride is typically poor when copper chloride is used alone. It is known that the activity of copper chloride can be improved in the oxyhydrochlorination process by use of promoters such as alkali metals and rare earth metals, such as potassium chloride. For example, U.S. Patent 2,575,167 describes a fluidized-bed process where cuprous chloride is supported on a finely divided solid. The inventor reports that an alkali metal chloride such as potassium chloride impregnated into the carrier along with cuprous chloride increases the yield of the process. U.S. Patent 2,498,546 claims a multi-step process employing a similar catalyst.

A problem early recognized with such processes as described by these patents is that of the stability of the catalyst. See, for example, U.S. Patent 3,657,367. The material $CuCl_2$ is generally considered to be the compound that reacts with oxygen to make chlorine available for the chlorination of methane. The material $CuCl_2$ can be volatilized at typical oxyhydrochlorination reaction temperatures and can also be reduced to the less reactive CuCl compound.

To counteract this loss of catalyst activity the use of alkali metals as stabilizing compounds has been suggested. The promoting and stabilizing of $CuCl_2$ in oxyhydrochlorination processes are reviewed in Allen et al., Rev. Pure and Appl. Chem 21:145-166 (1971). In spite of the stabilization considered to be provided by such compounds, it has generally been found necessary by those skilled in the art to apply high copper loadings to carrier materials to provide catalysts with acceptable sustainable activity. These high copper loadings on the carrier material create catalyst with high activity that are generally considered unsuitable for use in a commercial fixed-bed process. The high catalytic activity, coupled with the exothermic nature of the process, makes temperature control within the fixed-bed difficult. Lack of temperature control in the fixed-bed can result in temperatures that favor combustion processes, as described above, and greatly reduce process yield of the desired methyl chloride. To prevent temperature build up in fixed beds, it is generally considered necessary to provide feed materials to the fixed-bed at gas-fluid velocity rates that would provide product at a rate that would be uneconomical for a commercial reactor.

Unexpectedly, we have found a supported catalyst that has a high selectivity for methyl chloride that is stable at desired reaction temperatures and that has an activity suitable for use in a fixed-bed. This catalyst surprisingly comprises a level of copper previously thought too low to provide for sustainable catalytic activity in a fixed-bed. The catalyst is especially useful as a fixed-bed in a process conducted at a gauge pressure within a range of 0 to 689.5 kPa (0 psi to 100 psi) and a temperature within a range of 200°C. to 400°C. Within this pressure range a comparable yield of methyl chloride can be obtained at a lower temperature, when compared to a process ran at lower pressures. Operating the process within the desired pressure range also improves specificity of the process for methyl chloride. The ability to run the process at lower temperature while maintaining a desired conversion of methane also results in the production of less combustion products and in greater stability of the catalyst.

In our process, a gaseous mixture comprising methane, oxygen and hydrogen chloride is contacted with a fixed-bed of a catalyst. The fixed-bed of catalyst is formed in standard reactors suitable for containing such beds and allowing gas to pass through. Because of the corrosive nature of hydrogen chloride, it is generally preferred that the reactor be formed from materials known in the art to resist such corrosion.

To optimize the yield of methyl chloride and to avoid combustion products, it is important that the reactor for the process have good heat-transfer properties. For example, the catalyst is packed in the tubes of a multi-tube heat exchanger and a heat exchange fluid is then circulated through the shell side of the exchanger to maintain a constant temperature.

Next, methane, oxygen and hydrogen chloride are fed to the reactor. These three gases can be fed to the reactor individually or as a mixture. The methane is fed to the reactor as methane ($CH_4$) or as natural gas. When natural gas is used as a methane source, it is preferred that the natural gas be pretreated to remove moisture and sulfur compounds. Such pretreatment is effected by, for example, the use of appropriate zeolites. The source of oxygen fed to the reactor is elemental oxygen or air. It is also preferred that moisture be removed from the source of oxygen prior to feed to our process. Anhydrous hydrogen chloride is also fed to our process. In addition, other gases that are inert, such as neon, nitrogen, argon and the like, may be fed to the reactor along with the aforementioned reactants.

The molar ratio of methane to oxygen to hydrogen chloride ($CH_4:O_2:HCl$) used in our claimed process is varied within a wide range. However, it is generally preferred that the ratio of $CH_4:O_2:HCl$ be at least stoichiometric, i.e. 2:1:2. To assure optimal utilization of the hydrogen chloride and to promote methyl chloride selectivity, it is more preferred that our process be run with a stoichiometric excess of methane and oxygen. Therefore, it is preferred that the process be run at a molar ratio of methane to hydrogen chloride within a range of three to eight moles of methane for each mole of hydrogen chloride. It is also preferred that the claimed process be run with a molar excess of oxygen with respect to hydrogen chloride. The amount of excess of oxygen is not critical to our process and can be any such amount that does not result in excessive dilution of reactants and product. Generally, 0.5 to five moles of oxygen ($O_2$) per mole of hydrogen chloride is considered useful.

The present process requires a fixed-bed of a catalyst comprising a support selected from alumina or pyrogenic silica and, retained on the support, from 0.3 to 5 weight percent copper, from 5 to 25 weight percent lanthanum and from 0.1 to 5 weight percent lithium.

The pyrogenic silica useful as a support for the present catalyst, also known as fumed silica, is that formed by the thermal decomposition of silanes. The pyrogenic silica can have a BET surface area within a range of 200 $m^2$/g to 500 $m^2$/g. Preferred is when the pyrogenic silica has a BET surface area within a range of 350 $m^2$/g to 450 $m^2$/g.

The alumina can be any of those porous aluminas generally known in the art as "activated alumina," "gamma-alumina," and the like, made from the calcining of aluminum hydroxide precursors. The alumina can have a BET surface area within a range of 100 m$^2$/g to 300 m$^2$/g. Preferred is when the alumina has a BET surface area within a range of 150 m$^2$/g to 250 m$^2$/g.

A preferred support for the present process is alumina having a BET surface area within a range of 170 m$^2$/g to 220 m$^2$/g.

The catalyst of our invention has retained on the support 0.3 to 5 weight percent copper, 5 to 25 weight percent lanthanum and 0.1 to 5 weight percent lithium. The method of retaining these metals on the support is not critical to the present process and can be any method which provides for suitable retention of the metals under process condition. In a preferred method for forming our catalyst, a compound of the metal is placed in solution in an appropriate liquid media. The liquid media can be aqueous or nonaqueous media depending upon the solubility of the compound of the metal. The media preferably is, for example, water, tetrahydrofuran or acetonitrile. The support is then contacted with the media containing the compound of the metal to effect retention of the metal compound.

Separate solutions of compounds of copper, compounds of lanthanum and compounds of lithium can also be formed and contacted sequentially with the support. In the case of sequential contact of metal solutions with the support, it is preferred that the solution of the copper compound be contacted with the support first, followed by contact with the solution of lanthanum compound; or a solution mixture of lanthanum compound and lithium compound. Alternatively, one or more of the compound of lanthanum and compound of lithium may be placed in solution with the compound of copper and the resulting solution contacted with the support.

Compounds of copper, lanthanum and lithium which may be useful in the present process include metal oxides, metal hydroxides, metal halides, metal nitrates, metal sulfates, metal hydrides and organometallic complexes. The metal compounds include CuCl, CuCl$_2$, CuO, Cu$_2$O, {Cu(CH$_3$CN)$_4$}{PF$_6$}, LaCl$_3$, La(OH)$_3$, La(C$_2$H$_3$O$_2$)$_3$.xH$_2$O, LiCl, LiBr, LiOH, Cu(NO$_3$)$_2$.6H$_2$O, LaCl$_3$.7H$_2$O and mixed lanthanide chlorides.

A preferred catalyst for use in our process is alumina having retained thereon {Cu(CH$_3$CN)$_4$}{PF$_6$}, LaCl$_3$ and LiCl.

The catalyst useful in the present process comprises a support and retained on the support 0.3 to 5 weight percent copper, 5 to 25 weight percent lanthanum and 0.1 to 5 weight percent lithium, all based on the weight of catalyst. It is understood that the copper, lanthanum and lithium may be retained upon the support in the form of one or more compounds as described above. The weights of the copper, lanthanum and lithium on the support are based upon the weight of the elemental metal. It is preferred that the catalyst comprise a support and retained on the support from 0.5 to 3 weight percent copper, 10 to 20 weight percent lanthanum and 0.5 to 2 weight percent lithium.

The methane, oxygen and hydrogen chloride are contacted in our process with the catalyst at a temperature within a range of 200°C. to 400°C. A preferred temperature is within a range of 300°C. to 380°C. Even more preferred is a temperature within a range of 315°C. to 340°C.

The present process is conducted at a gauge pressure within a range of 0 to 689.5 kPa (0 to 100 psi). Preferred is when the process is conducted at a pressure within a range of 68.9 to 413.7 kPa (10 to 60 psi). Most preferred is a pressure within a range of 137.9 to 275.8 kPa (20 to 40 psi). The present inventors have unexpectedly found that within these preferred pressure ranges a surprisingly improved ratio of methyl chloride to dichloromethane is achieved. Also, within these preferred pressure ranges, it is possible to run our claimed process at a lower temperature thereby reducing combustion products and thermal decomposition of the catalyst.

<u>Example 1</u> (Comparison example)

A particulate catalyst having a high loading of copper was evaluated as an oxyhydrochlorination catalyst for forming methyl chloride. The catalyst comprised by weight 27.7% Cu, 4.98% K and 3.95% La supported on silica. The catalyst was obtained from Pittsburgh Energy Technology Center, Pittsburgh, PA and was described as being prepared by a method similar to that described in C. E. Taylor et al., **Methane Conversion,** 1988, Elsevier Science, Amsterdam, The Netherlands, p. 483-489. A similar procedure for making such a catalyst is described in U.S. Patent 4,769,504.

Then 4.2 g of the catalyst was packed in a 0.6 cm inside diameter quartz glass tube (hereafter referred to as the "reactor"), which was then placed in a three-zone furnace equipped with a temperature controller. The catalyst was pretreated by heating at 290°C. for one hour in the presence of hydrogen gas.

The reactor was then heated to a stabilized temperature of 315°C. and feed of the reactants was started. Reactants fed to the reactor were Linde Gas (Villawood, NJ) USP grade O$_2$, technical grade HCl and a certified standard mixture of 2% neon in CH$_4$ (Matheson Gas Products, Twinsburg, OH). The rate of feed of reactants to the reactor was methane at 100 cm$^3$/min., oxygen at 22.8 cm$^3$/min. and HCl at 29.6 cm$^3$/min. The gas velocity through the reactor was 9 cm/s and the run time was 136 hours.

During the run, the temperatures of the reactor and catalyst bed were monitored by thermocouples attached to the wall of the reactor and positioned within the catalyst bed. At 30 hours after initiation of feed to the reactor, a significant exotherm within the reactor bed was evidence by the temperature rapidly rising from 315°C. to 550°C. The temperature subsequently returned to a steady level of 350°C. Initially the conversion of methane in the reactor was 27%, however, at 75 hours into the run the conversion of methane dropped to 12 percent where it remained for the remainder of the 136 hour run. These results demonstrate the typical exotherm and loss of catalyst activity that can occur with a catalyst having a high copper loading.

Example 2 (Comparison example)

A catalyst comprising 2.5 weight percent copper on silica was evaluated in an oxyhydrochlorination process for forming methyl chloride. The silica was fumed silica, obtained from PQ Corp., Lafayette Hill, PA, having a surface area of 380 $m^2$/g. The silica was dried by heating at 400°C. under vacuum. A solution comprising 8.75 g of {Cu($CH_3CN)_4$}{$PF_6$} in 150 ml of acetonitrile was prepared. Then 50 g of silica were placed in this solution for 16 hours at room temperature. The treated silica was dried with flowing nitrogen and further dried at 350°C. for two hours under vacuum. The silica was determined by Inductive Coupled Plasma Atomic Emission Spectroscopy (ICP-AES) to comprise 2.5 weight percent copper. Next, 2.9 g of this catalyst were packed in a reactor as described in Example 1. The catalyst was pretreated by heating at 300°C. for one hour and then at 340°C. for one hour under flowing nitrogen. The reactor was brought to a temperature of 340°C. for the first 90 hours of the run and then heated to 350°C. for the remainder of the run. Reactants fed to the reactor were as described in Example 1. The rate of feed of reactants to the reactor were methane at 100 $cm^3$/min., oxygen at 22.8 $cm^3$/min. and HCl at 29.6 $cm^3$/min. The gas velocity through the reactor was 9 cm/s and the length of the run was 290 hours. No exotherm, as observed in Example 1, was observed in this run. The gas exiting the reactor was analyzed by gas chromatography using a thermal conductivity detector (GC-TCD). The conversion of methane was calculated as a percent of the total methane fed to the process. The conversion of methane when the temperature was at 340°C. was four percent and the conversion at 350°C. was five percent. The results demonstrate the poor methane conversion associated with a non-promoted catalyst having a low copper loading.

Example 3 (Comparison example)

A catalyst comprising 1.8 weight percent copper and 10.2 weight percent lanthanum on silica was evaluated in an oxyhydrochlorination process for forming methyl chloride.

Copper chloride was retained on the silica by a method similar to that described in Example 2. A solution comprising 6.7 g of $LaCl_3$ in 50 ml of water was prepared and 12.9 grams of the catalyst prepared in Example 2 was placed in this solution for 16 hours at room temperature. The treated silica was dried at 100°C. under flowing nitrogen and then further dried at 330°C. for three hours under vacuum. The treated silica was determined by ICP-AES to comprise 1.8 weight percent copper and 10.2 weight percent lanthanum. Then 4.1 g of this catalyst were packed in a reactor as described in Example 1. The catalyst was pretreated by heating for 0.5 hour at 310°C. and then for 1.5 hours at 340°C. under flowing nitrogen. The reactor was maintained at a constant temperature of 340°C. during the course of the 265 hour run. Reactants fed to the reactor were as described in Example 1. The rate of feed of reactants to the reactor were methane at 100 $cm^3$/min., oxygen at 22.8 $cm^3$/min. and HCl at 29.6 $cm^3$/min. The gas velocity through the reactor was 9 cm/s. The gas exiting the reactor was analyzed by GC-TCD and methane conversion calculated as previously described. Initially, the conversion of methane within the reactor was within a range of 15 to 18 percent. However, by ten hours into the run, a gradual decline in conversion of methane was observed. The total conversion of methane for the 265 hour run was eight percent. The results demonstrate the transitory ability of lanthanum to improve the copper catalyzed oxyhydrochlorination of methane.

Example 4 (Comparison example)

A catalyst comprising 2.6 weight percent copper and 1.3 weight percent lithium on silica was evaluated in an oxyhydrochlorination process for forming methyl chloride.

Copper was retained on the silica by a method similar to that described in Example 2. A solution comprising 1.08 g of LiCl in 100 ml of water was prepared and 12.9 grams of the catalyst from Example 2 was placed in the solution for 16 hours at room temperature. The treated silica was then dried under flowing nitrogen at room temperature and then at 330°C. Next, 2.9 g of this catalyst were then packed in a reactor as described in Example 1. The catalyst was pretreated by heating at 340°C. for 1.5 hours under flowing nitrogen. The reactor was maintained at a constant temperature of 340°C. during the course of a 160 hour run. Reactants fed to the reactor were as described in Example 1. The rate of feed of reactants to the reactor were methane at 100 $cm^3$/min., oxygen at 22.8 $cm^3$/min. and HCl at 29.6 $cm^3$/min. The gas velocity through the reactor was 9 cm/s. The gas exiting the reactor was analyzed by GC-TCD and methane conversion calculated as previously described. The total conversion of methane for the 160 hour run was six percent. This catalyst did not show the initial high activity observed with the silica supported copper and lanthanum catalyst described in Example 3, but showed only minor loss of activity over the period of the run.

Example 5

A catalyst comprising 1.5 weight percent copper, 13.5 weight percent lanthanum and 0.7 weight percent lithium on silica was evaluated in an oxyhydrochlorination process for forming methyl chloride.

The copper was retained on the silica by a method similar to that described in Example 2. The silica (12.9 g) containing the copper was then treated with a solution comprising 1.08 g LiCl and 6.72 g $LaCl_3$ in 100 ml of water by procedures similar to those described in Examples 3 and 4. The treated silica was determined by ICP-AES to comprise 1.5 weight percent copper, 13.5 weight percent lanthanum and 0.7 weight percent lithium. Then, 3.8 g of this catalyst were packed in a reactor as described in Example 1. The catalyst was pretreated by heating at 340°C. for 1.5 hours under flowing nitrogen. The reactor was maintained at a constant temperature of 340°C. during the course of a 210 hour run. Reactants fed to the reactor were as described in Example 1. The rate of feed of reactants to the reactor were methane at 100 $cm^3$/min., oxygen at 22.8 $cm^3$/min. and HCl at 29.6 $cm^3$/min. The gas velocity through the reactor was 9 cm/s. The gas exiting the reactor was analyzed by GC-TCD and the conversion of methane calculated as previously described. Initial conversion of methane in the reactor was within a range of 17 to 20 percent and the average conversion of methane for the run was 16 percent. No exothermic temperature spike, as observed in Example 1, was observed.

Example 6

A catalyst having 1.1 weight percent copper, 18.4 weight percent lanthanum and 0.8 weight percent lithium on silica was evaluated in an oxyhydrochlorination process for forming methyl chloride.

The copper was retained on the silica by a method similar to that described in Example 2. A solution comprising 13.02 g of $LaCl_3$ and 2.09 g of LiCl in 100 ml of water was prepared. Then, 25 grams of the silica containing the copper was contacted with this solution for 16 hours at room temperature. The treated silica was dried at room temperature under flowing nitrogen. The treated silica was determined by ICP-AES to comprise 1.1 weight percent copper, 18.4 weight percent lanthanum and 0.8 weight percent lithium. Next, 4.3 g of this catalyst were packed in a reactor as described in Example 1. The catalyst was pretreated by heating at 340°C. for two hours under flowing nitrogen. The reactor was maintained at a temperature of 340°C. for the duration of a 93 hour run. Reactants fed to the reactor were as described in Example 1. The rate of feed of reactants to the reactor were methane at 100 $cm^3$/min., oxygen at 22.8 $cm^3$/min. and HCl at 29.6 $cm^3$/min. The gas velocity through the reactor was 9 cm/s. The gas exiting the reactor was analyzed by GC-TCD and methane conversion calculated as previously described. Initial conversion of methane within the reactor was 22 percent, with total conversion for the run being 20 percent.

Example 7

The effects of pressure and temperature on an oxyhydrochlorination process using a catalyst comprising copper, lanthanum and lithium on silica were also evaluated.

The catalyst was prepared and pretreated by methods similar to those described in Example 6 and comprised 1.1 weight percent copper, 18.4 weight percent lanthanum and 0.8 weight percent lithium. The reactor was a Hastelloy™ C tube having an inside diameter of 0.44 cm. Next, 1.7 g of catalyst were added to the reactor as a packed-bed. Reactants fed to the reactor were as described in Example 1. The rate of feed of reactants to the reactor were methane at 80 $cm^3$/min., oxygen at 13.3 $cm^3$/min. and HCl at 17.3 $cm^3$/min. Nitrogen was fed to the reactor at a rate of 63.3 $cm^3$/min. The gauge pressure of the reactor was maintained at 206.8 to 275.8 kPa (30 or 40 psi) as described in Table 1.

The initial temperature of the reactor was 320°C. and this was periodically increased as described in Table 1. At each temperature described in Table 1, the percent conversion of methane was determined as previously described (%$CH_4$ Conv.) as well as the percent of carbon monoxide (%CO) and carbon dioxide (%$CO_2$) formed. The results for carbon monoxide and carbon dioxide are reported as the area percent under the GC-TCD trace. The percent $CH_3Cl$ and $CH_2Cl_2$ are also reported as the area percent under the GC-TCD trace.

Table 1

| Effects of Temperature and Pressure on Oxyhydrochlorination Process Employing a Silica Supported Cu, La and Li Catalyst | | | | | | |
|---|---|---|---|---|---|---|
| Gauge Press. (psi) | Temp. (°C.) | %$CH_4$ Conv. | %$CH_3Cl$ | %$CH_2Cl_2$ | %CO | %$CO_2$ |
| 30* | 320 | 11 | 88 | 12 | 0.0 | 0.0 |
| 30 | 325 | 13 | 85 | 13 | 0.0 | 1.0 |
| 30 | 330 | 17 | 80 | 17 | 0.0 | 3.0 |
| 30 | 335 | 17 | 81 | 15 | 0.0 | 3.0 |
| 40** | 315 | 15 | 85 | 13 | 0.0 | 2.0 |
| 40 | 320 | 17 | 78 | 16 | 3.0 | 3.0 |

*30 psi = 206.8 kPa
**40 psi = 275.8 kPa

## Example 8

A catalyst comprising 0.9 weight percent copper, 18.8 weight percent lanthanum and 0.8 weight percent lithium on gamma alumina was evaluated in an oxyhydrochlorination process for forming methyl chloride. The gamma alumina was obtained from United Catalysts, Inc., Louisville, KY, and had a surface area of 188 $m^2$/g. A solution comprising 4.40 g of {Cu(CH$_3$CN)$_4$}{PF$_6$} in 100 ml of tetrahydrofuran (THF) was formed. Then, 25 g of the alumina were placed in this solution for 16 hours at room temperature. The treated alumina was then dried under flowing nitrogen. A solution comprising 13.02 g of LaCl$_3$ and 2.09 g of LiCl in 150 ml of water was prepared and 25 g of the alumina containing copper was contacted with the solution for 16 hours at room temperature. The treated alumina was first dried at room temperature under flowing nitrogen and then at 330°C. for 3 hours under vacuum. The treated alumina was determined by ICP-AES to comprise 0.9 weight percent copper, 18.8 weight percent lanthanum and 0.8 weight percent lithium.

Next, 7.5 g of the catalyst were packed in a reactor. The catalyst was pretreated by heating at 340°C. for 1.5 hours under flowing nitrogen. Reactants fed to the reactor were as described in Example 1. The rate of feed of reactants to the reactor were methane at 100 $cm^3$/min., oxygen at 22.8 $cm^3$/min. and HCl at 29.6 $cm^3$/min. The gas velocity through the reactor was 9 cm/s.

The initial temperature of the reactor was 340°C. and this was periodically decreased as described in Table 2. At each temperature given in Table 2, gas exiting the reactor was analyzed by GC-TCD and the percent conversion of methane (%$CH_4$ Conv.) calculated as previously described. The percent of carbon monoxide (%CO), carbon dioxide (%$CO_2$), $CH_3Cl$ and $CH_2Cl_2$ formed are reported in Table 2 as the area percent under the GC-TCD trace.

Table 2

| Effects of Oxyhydrochlorination Catalyst Comprising Cu, La and Li on Alumina | | | | | |
|---|---|---|---|---|---|
| Temp. °C | %$CH_4$ Conv. | %$CH_3Cl$ | %$CH_2Cl_2$ | %CO | %$CO_2$ |
| 315 | 11 | 77 | 15 | 0 | 6 |
| 320 | 13 | 74 | 17 | 0 | 7 |
| 325 | 16 | 71 | 18 | 0 | 7 |
| 330 | 19 | 66 | 20 | 0 | 10 |
| 335 | 21 | 61 | 20 | 1 | 13 |
| 340 | 22 | 56 | 20 | 2 | 17 |

## Claims

1. A catalyst composition for oxyhydrochlorination of methane, the composition comprising: a support selected from alumina or pyrogenic silica and, retained on the support, from 0.3 to 5 weight percent copper, from 5 to 25 weight percent lanthanum and from 0.1 to 5 weight percent lithium.

2. A process for oxyhydrochlorination of methane, the process comprising: contacting methane, oxygen and hydrogen chloride with a fixed-bed of the catalyst according to claim 1 at a temperature within a range of 200°C. to 400°C. and at a gauge pressure of 0 to 689.5 kPa (0 to 100 psi).

3. A process according to claim 2 where the catalyst is packed in the tubes of a multi-tube heat exchanger reactor and a heat exchange fluid is circulated through the shell side of the exchanger to control the temperature of the process.

4. A process according to claim 2 where the methane and oxygen are fed to the process in at least stoichiometric amounts relative to the hydrogen chloride.

5. A process according to claim 2 where the process is run with a stoichiometric excess of methane and oxygen relative to the hydrogen chloride.

6. A process according to claim 5 where three to eight moles of methane and 0.5 to five moles of oxygen are fed to the process per each mole of hydrogen chloride fed to the process.

7. A process according to claim 2 where the support is pyrogenic silica having a surface area within a range of 200 $m^2$/g to 500 $m^2$/g.

8. A process according to claim 2 where the support is alumina having a surface area within a range of 100 $m^2$/g to 300 $m^2$/g.

9. A process according to claim 2 where the catalyst essentially consists of alumina having retained thereon $\{Cu(CH_3CN)_4\}\{PF_6\}$, $LaCl_3$ and $LiCl$.

10. A process according to claim 2 where three to eight moles of methane and 0.5 to five moles of oxygen are fed to the process per each mole of hydrogen chloride fed to the process, the support is alumina having a surface area within a range of 170 $m^2$/g to 220 $m^2$/g, the support has retained thereon 0.1 to 3 weight percent copper, 10 to 20 weight percent lanthanum and 0.5 to 2 weight percent lithium based on the weight of the catalyst, the temperature is within a range of 315°C. to 340°C. and the process is conducted at a gauge pressure within a range of 137.9 to 275.8 kPa (20 to 40 psi).

**European Patent Office**

# EUROPEAN SEARCH REPORT

Application Number

EP 96 30 0056

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | PREPR. - AM. CHEM. SOC., DIV. PET. CHEM. (ACPCAT,05693799);92; VOL.37 (1); PP.281-3, DEP. ENERGY;PITTSBURGH ENERGY TECHNOL. CENT.; PITTSBURGH; 15236-0940; PA; USA (US), XP 000568413 NOCETI R P ET AL 'Advances in methane oxyhydrochlorination catalysts' * the whole document * | 1,2 | C07C17/154 C07C19/03 B01J23/83 |
| A | BE-A-645 772 (SICEDISON) * claims; example 6 * | 1 | |
| A | US-A-2 957 924 (T.W. HEISKELL ET AL) * the whole document * | 3 | |

TECHNICAL FIELDS SEARCHED (Int.Cl.6)

C07C
B01J

The present search report has been drawn up for all claims

| Place of search | Date of completion of the search | Examiner |
|---|---|---|
| THE HAGUE | 15 April 1996 | Bonnevalle, E |

EPO FORM 1503 03.82 (P04C01)